# EUROPEAN PATENT APPLICATION

(11) **EP 1 607 099 A1**
(43) Date of publication of application: **21.12.2005**
(21) Application number: 05015870.8
(22) Date of filing: 04.06.1999
(51) Int. Cl.: A61K 35/78

(54) **Use of hyperforin derivatives as an antidepressant agent**

(30) Priority: 10.06.1998 IT MI981312
(62) Divisional of application: 99926516.8
(71) Applicant: INDENA S.p.A., 20139 Milano (IT)
(72) Inventor: Bombardelli, Ezio, 27027 Groppello Cairoli (PV) (IT); Morazzoni, Paolo, 20139 Milano (IT)
(74) Representative: Banfi, Paolo

(57) **Abstract**

Hyperforin derivatives of formula I

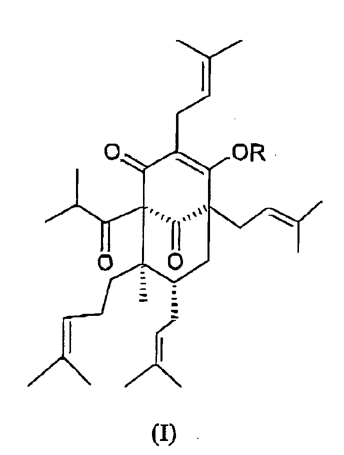

wherein R is an acyl or a glicoside group, have advantageous pharmacological characteristics and good stability, and are used for the preparation of an antidepressive medicament.

## Description

The present invention relates to the use of hyperforin derivatives for the treatment of depression and anxiety.

Flowering tops of Hypericum perforatum contain a number of classes of structurally different substances acting directly or indirectly on central nervous system.

More particularly, said compounds comprise hypericin, hyperforin, and dimeric flavones which exert antidepressive and anxiolytic activities on animals and humans.

The action mechanisms of these compounds are different: anti-MAO action, action on serotonin release, and benzodiazepine-like activity.

Hyperforin, which is one of the main components of the lipophilic fraction of Hypericum perforatum flowering tops, has recently been the object of numerous studies which made it possible to establish its important role as antidepressant; studies carried out by the Applicant proved that this molecule has serotonin-mimetic activity.

Hyperforin is not very stable in the usual conditions of extraction and conservation; according to WO 97/13489 (Schwabe), the hyperforin content in a water-alcoholic extract of St.-John's-wort falls nearly to zero already after a few weeks.

Again according to WO 97/13489, in order to obtain stable extracts with a constant content in hyperforin, extraction, purification and conservation should be carried out in the presence of antioxidants such as vitamin C and the esters thereof, sulfated amino acids, etc.

The high instability of hyperforin makes therefore the preparation of formulations rather difficult.

The hyperforin derivatives according to the invention, which are stable and more active as antidepressants in specific pharmacological tests, have the following formula I: 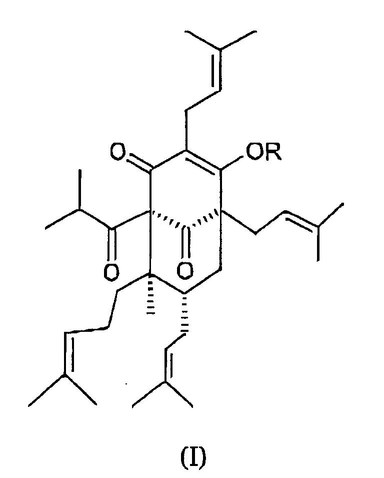 in which R is:
- a saturated or unsaturated, straight or branched, C₁-C₂₂ acyl group, optionally having one or more substituents, which can be the same or different, selected from halogen atoms, nitro, amino, C₁-C₆-alkylamino, di- C₁-C₆-alkylamino, C₁-C₆-acylamino groups;
- a cycloaliphatic or aromatic acyl residue in which the aromatic moiety has optionally one or more substituents, which can be the same or different, selected from halogen atoms, hydroxy, methoxy, amino groups;
- a glycidic residue in which one or more hydroxy groups are optionally alkylated or acylated.

"Aromatic acyl residue" preferably means a benzoyl or cinnamyl residue having one or more amino or alkoxy groups.

"Glycidic residue" means the residue of one sugar bound by an ether bond to the hydroxy group at the 1- position of the pyranosyl or furanosyl ring, being the other hydroxy groups of the sugar optionally methylated or acetylated.

Preferred R groups are acetyl, monochloroacetyl, butyryl, γ-aminobutyryl, p-aminobenzyl, trimethoxybenxyl, trimethoxycinnamyl, β-glucosyl and β-galactosyl.

The hyperforin derivatives of the invention can be prepared with conventional methods for the acylation or glicosylation of hydroxy groups.

For example, hyperforin substantially pure or an extract enriched in hyperforin can be subjected to reaction with acid chlorides or anhydrides of RCOOH acids (R as defined above) in suitable solvents, such as pyridine.

On the other hand, glicosylation can be carried out using a suitably protected reactive derivative of the desired sugar (ROH), for example α-D-glucopyranosyl bromide tetraacetate.

According to a particularly convenient aspect of the invention, compounds I are prepared by extracting Hypericum perforatum flowering tops with carbon dioxide in supercritical conditions, subsequently partitioning between solvents and derivatizing hyperforin in the resulting extract.

Leaves and flowering tops of St.-John's-wort, separately or in mixture, mainly as natural mixture, are extracted with carbon dioxide in supercritical conditions under pressures ranging from 180 to 260 bars, preferably 240 bars and at temperatures ranging from 35 to 50°C, preferably 40°C. A lipophilic extract is obtained containing about 50% of hyperforin. The extract contains considerable amounts of xanthones, waxes, fatty acids and triglycerids. The Hyperforin percentage is subsequently increased, according to a further aspect of the invention, solubilizing the resulting extract in methanol or in partially aqueous acetonitrile and extracting then the solution with n-hexane or aliphatic hydrocarbons. The hydrocarbon phase contains undesired substances which are removed; the hydrophilic phase is diluted with about an equal volume of water and with aliphatic hydrocarbons. The extract of St.-John's-wort obtained by concentration of the lipophilic phase can be used for the preparation of the derivatives as described above.

The derivatives of the invention exert no activity *in vitro* on receptors, while being particularly active *in vivo*, exerting a dose-related strong antidepressive activity.

In an *in vivo* test in mice and rats, the compounds of the invention have shown a higher activity than hyperforin and Hypericum ethanolic or methanolic extracts.

As *in vivo* test to verify the antidepressive effect, were selected the escape deficit development test and the inhibition of the ethanol consumption in Sardinia alcohol preferring rats according to models known in literature.

In the escape deficit development test, the compounds of the invention have shown a higher activity than the known extracts and an activity comparable with that of known medicaments such as imipramine. In this test, rats are fastened and subjected to mild, short, unavoidable electric shocks for 50 min (pre-test). Twenty-four hours later, animals are tested for their ability to avoid the same stimuli on their tails, in a situation in which escape is impossible. A rat on the average makes 26 escapes out of 30 stimuli (naive controls), whereas an animal subjected to pre-test only makes 1-3 escapes (ED controls). Hyporeactivity induced by the pre-test does not take place in rats pre-treated for 1-3 weeks with antidepressants such as imipramine or fluoxetine. The compounds of the invention administered to rats one hour before exposure to the unavoidable stress cause an increase in reactivity to the escape test, which is enhanced when pre-treatment is effected for 1-2 weeks.

For example, treatment of rats with compound I in which R is acetyl yields the results reported in the following Table:

**Table -**

| Antidepressive effect of Hyperforin acetate in rats in the escape test with a 2 week- pre-test | | |
|---|---|---|
| Substances | Dose mg/kg | Number of escapes |
| Hyperforin acetate | 6.25 | 12.6 ± 2.8 |
| Hyperforin acetate | 12.5 | 17.3 ± 1.9 |
| Hyperforin acetate | 25.0 | 21.2 ± 1.3 |
| Hyperico alcoholic extract | 1000 | 15.6 ± 2.4 |
| Hyperico hexane extract | 600 | 16.9 ± 1.2 |
| Ed controls | | 2.6 ± 0.7 |
| Naive controls | | 23.6 ± 1.2 |
| Statistical analysis: Kruskal-Wallis non parametric ANOVA KW = 13.462 p = 0.0012 | | |
| Hypericum alcoholic extract and hexane extract vs naive | | p<0.01 |
| Hyperforin acetate 25 mg vs naive | | n.s. |
| Naive vs AND | | p<0.01 |

In the test for the reduction of alcohol consumption in Sardinia rats (which is an index of depression and anxiety) according to procedures known in literature, the products of the invention induce, after two-day administration, a 60 to 75% decrease in alcohol consumption in favour of water compared with controls.

The compounds of formula I can be formulated in soft-gelatin capsules, hard-gelatin capsules, tablets, suppositories; preferably the compounds of the invention are formulated in soft-gelatin capsules or in controlled-release formulations. The dosages of compounds in the formulations range from 5 to 50 mg per dose in the usual formulations and up to 200 mg in the controlled-release formulations, in this case the preferred dose being 200 mg per dose daily.

The example reported hereinbelow illustrate the invention in greater detail.

### Example 1 - Preparation of a Hyperforin-enriched extract

10 kg of biomass of *Hypericum perforatum* are extracted according to the procedure reported below, in a 25 L extraction plant for supercritical gas, equipped with two separators.

10 kg of *Hypericum perforatum* flowering tops mechanically dried, after collecting, at a temperature not above 60°C, are extruded into cubes so as to break cells and extracted with CO₂ in supercritical conditions under the following experimental conditions:
- temperature: 45°C in the extractor, 30°C in the first separator and 20°C in the second separator;
- pressure: 240 bars in the extractor, 100 bars in the first separator and 50 bars in the second separator.

The CO₂ flow was 10 L per minute for 45 minutes. The extract was concentrated in the second separator, whereas most water present in the vegetable matrix was concentrated in the first separator. The extract present in the second separator is solubilised in 3.2 L of methanol and this solution is extracted with 3 x 1.5 L of n-hexane.

The hexane phase is counter-washed with 98% methanol using hyperforin as marker which should remain in the methanol phase. The hexane phase is removed whereas the combined metanolic ones are diluted with 0.6 L of water and re-extracted with 2 x 0.6 L of n-hexane.

The combined hexane phases are decolourized with 0.3% charcoal, dried over Na₂SO₄, then concentrated under vacuum at a temperature not above 40°C to an oil. 0.22 kg of a waxy extract are obtained, having an about 70% hyperforin content.

## Claims

1. The use of hyperforin derivatives of formula (I): 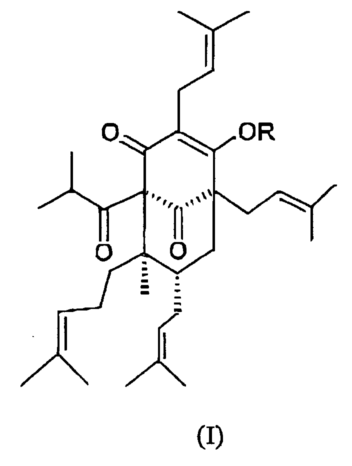 in which R is:
- a saturated or unsaturated, straight or branched, C₁-C₂₂ acyl group, optionally having one or more substituents, which can be the same or different, selected from halogen atoms, nitro, amino, C₁-C₆-alkylamino, di- C₁-C₆-alkylamino, C₁-C₆-acylamino groups;
- a glycidic residue in which one or more hydroxy groups are optionally alkylated or acylated,
for the preparation of an antidepressive medicament.

2. The use according to claim 1, wherein in formula (I) R is selected from butyryl, γ-aminobutyryl, β-glucoside and β-galactosyl.
